# EUROPEAN PATENT APPLICATION

(11) **EP 1 652 474 A2**
(43) Date of publication of application: **03.05.2006**
(21) Application number: 05256673.4
(22) Date of filing: 27.10.2005
(51) Int. Cl.: A61B 5/15

(54) **Combined lancing and auxiliary device**

(30) Priority: 28.10.2004 US 975234
(71) Applicant: LifeScan, Inc., Milpitas, CA 95035 (US)
(72) Inventor: Olson, Lorin P., Scotts Valley, CA 95066 (US); Leong, Koon-wah, Sunnyvale, CA 94086 (US); Talreja, Priya, Fremont, CA 94538 (US)
(74) Representative: Mercer, Christopher Paul

(57) **Abstract**

A combined lancing and auxiliary device (300) includes a lancing device (302) with a lancing auxiliary device (304) attached thereto. The lancing auxiliary device can be, for example, a compartment for securely and removably containing items associated or unassociated with lancing, a timer (504) or personal digital assistant device (604) for assisting in documentation, or an illumination device (404) for illuminating a target site. The lancing auxiliary device can be permanently or removably attached to the lancing device.

## Description

### BACKGROUND OF THE INVENTION

1. Field of the Invention

The present invention relates, in general, to medical devices and, in particular, to lancing devices.

2. Description of the Related Art

Conventional lancing devices generally have a rigid housing, various operating mechanisms and a lancet that can be armed and launched so as to briefly protrude from one end of the lancing device. For example, conventional lancing devices can include a lancet that is mounted within a rigid housing such that the lancet is movable relative to the rigid housing along a longitudinal axis thereof. Typically, the lancet is spring loaded and launched, upon release of the spring, to penetrate (i.e., "lance") a target site (e.g., a dermal tissue target site). A bodily fluid sample (e.g., a whole blood sample) can then be expressed from the penetrated target site for collection and analysis. Conventional lancing devices are described in U.S. Patent No. 5,730,753 to Morita, U.S. Patent No. 6,045,567 to Taylor et al., U.S. Patent No. 6,071,250 to Douglas et al., U.S. Patent 6,156,051 to Schraga, and U.S. Patent 6,607,543 to Purcell et al., each of which is incorporated fully herein by reference.

Conventional lancing devices typically require a user to arm the lancing device, urge the lancing device against a target site, and then press a button or other switch to manually activate the lancing device such that a lancet within the device is launched (also referred to as "fired") towards the target site. The lancet then penetrates (e.g., lances) the target site, thereby creating an opening for the expression of a bodily fluid sample.

Use of lancing devices can be inconvenient and cumbersome due to the multitude of items associated with lancing that must be carried (e.g., lancets, test strips, alcohol swabs, and pharmaceuticals) as well as the need to document results of analyses conducted on the bodily fluid samples produced with the lancing device.

Still needed in the field, therefore, is a lancing device that that minimizes the inconvenience and cumbersomeness of carrying items associated with lancing. In addition, a lancing device that reduces the inconvenience of documentation is also desired.

### SUMMARY OF THE INVENTION

Combined lancing and auxiliary devices according to embodiments of the present invention minimize the inconvenience and cumbersomeness of carrying items associated with lancing. In addition, combined lancing and auxiliary devices according to embodiments of the present invention also reduce the inconvenience of documentation.

A combined lancing and auxiliary devices according to an embodiment of the present invention includes a lancing device with a lancing auxiliary device attached thereto. The lancing auxiliary device can be, for example, a compartment for securely and removably containing items associated or unassociated with lancing, a timer or personal digital assistant device for assisting in documentation, an illumination device for illuminating a target site or any combination thereof. The lancing auxiliary device can be permanently or removably attached to the lancing device. Attachment and detachment of the lancing auxiliary device makes the combined lancing and auxiliary device longer or shorter, respectively, for better ergonomic fit to different sized hands.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, of which:
FIG. 1 is a perspective view of a compact lancing device according to an exemplary embodiment of the present invention;
FIG. 2 is a perspective exploded view of the compact lancing device of FIG. 1;
FIG. 3 is a perspective exploded view of the lancet holder and trigger mechanism of the compact lancing device of FIG. 1;
FIGs. 4A and 4B are perspective views of the lancet holder and trigger mechanism of the compact lancing device of FIG. 1 in an armed position and in a triggered position, respectively (with dashed lines indicating features not visible in the view of FIGs. 4A and 4B);
FIGs. 5A and 5B are cross-sectional schematic views of the compact lancing device of FIG. 1 set at maximum and minimum lancet needle penetration depths, respectively;
FIG. 6 is a cut-away perspective view of the compact lancing device of FIG. 1;
FIGs. 7A through 7E are simplified, schematic, cross-sectional views depicting the compact lancing device of FIG. 1 during various stages of operation;
FIG. 8 is a perspective view of a combined lancing and auxiliary device according to an embodiment of the present invention;
FIG. 9 is partially cut away perspective view of the combined lancing and auxiliary device of FIG. 8;
FIG. 10 is a perspective view of a combined lancing and auxiliary device according to another embodiment of the present invention;
FIG. 11 is a perspective view of a combined lancing and auxiliary device according to still another embodiment of the present invention; and
FIG. 12 is a perspective view of a combined lancing and auxiliary device according to a further embodiment of the present invention.

### DETAILED DESCRIPTION OF THE INVENTION

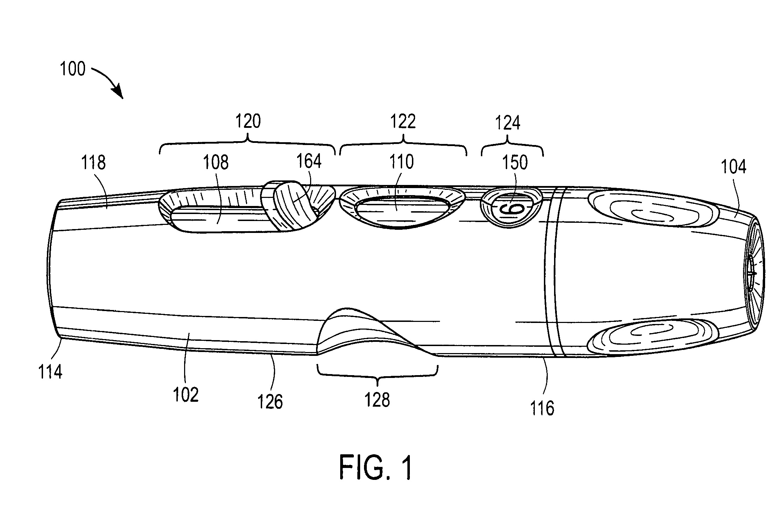

FIGs. 1, 2, 3, 4A, 4B, 5A, 5B and 6 are various depictions a compact lancing device 100 according to an embodiment of the present invention. Compact lancing device 100 includes a housing 102, end cap 104, depth adjustment mechanism 106, arming mechanism 108, trigger mechanism 110, and launching mechanism 112.

As described in detail below, launching mechanism 112, arming mechanism 108 and trigger mechanism 110 are operatively connected such that a target site (e.g., a user's skin target site) can be lanced with a lancet (for example, lancet L that includes lancet needle N) held within compact lancing device 100. In this regard, launching mechanism 112 is configured for launching lancet L such that lancet needle N lances a target site, arming mechanism 108 is configured for arming lancing device 100 prior to firing the compact lancing device (i.e., prior to launching lancet L), and trigger mechanism 110 is configured to actuate the firing of compact lancing device 100. Furthermore, depth adjustment mechanism 106 is configured for a user to select (i.e., predetermine) needle penetration depth into a target site.

Compact lancing device 100 can be any suitable size but is typically sized to fit within the palm of a user's hand and has, therefore, a typical but non-limiting length in the range of 50mm to 70mm and a typical but non-limiting width in the range of about 10mm to about 20 mm. Such a compact size is beneficial in that it requires less storage space and is less conspicuous than conventionally sized lancing devices.

Housing 102 is generally cylindrical in shape and includes a proximal end 114, a distal end 116, a first surface 118, an arming mechanism orifice 120, a trigger mechanism orifice 122, a window 124, a second surface 126 and a gripping feature 128. Housing 102 can be formed, for example, of rigid materials including, but not limited to, polycarbonate, polyester, polystyrene, polyamide, polyacetal, polyimide, polyketone, polyurethane, polybutyleneteraphthalate and combinations thereof. Housing 102 can also be formed of semi-rigid materials including, for example, polypropylene, high density polyethylene, polyurethane, ethylene propylene rubber, polymethylpentene and combinations thereof. If desired, housing 102 can be easily manufactured from two elongate pieces that are sonically welded or snap-fit together to create housing 102.

End cap 104 is detachably connected to distal end 116 of housing 102. End cap 104 includes skin engaging surface 130 with opening 132 therein, collar engaging end 134 with raised features 136, and indentations 138.

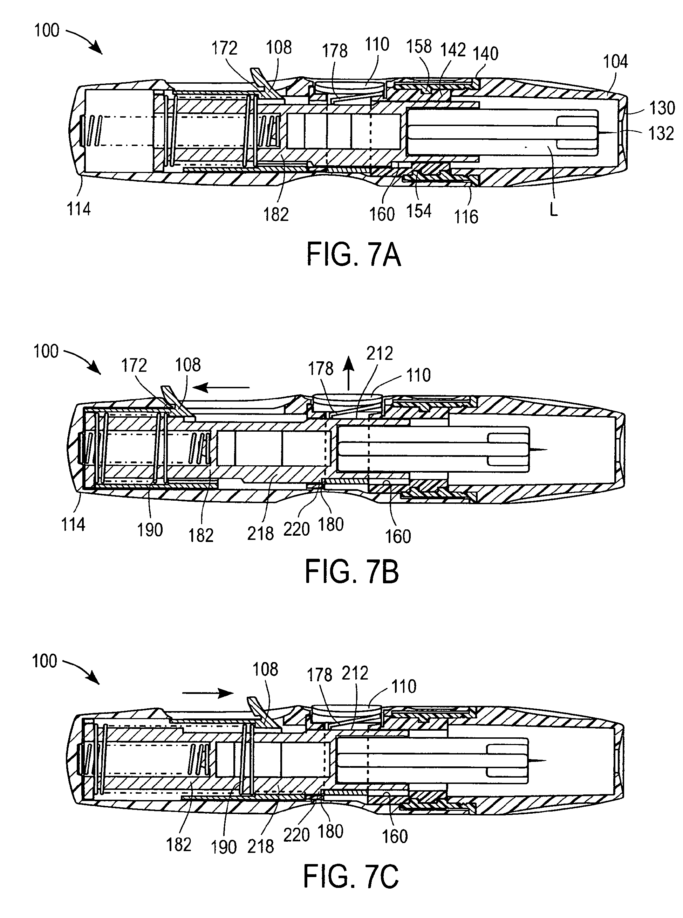

Depth adjustment mechanism 106 includes a collar 140 and a guide member 142. Collar 140 includes a collar first end 144 with collar rim 146, a collar second end 148, depth setting indicators 150, recesses 152 and internal spiral thread 154. Furthermore, guide member 142 includes an aperture 156, a cam surface 158, a guide member groove 160 (labeled in FIGs. 7A, 7B and 7C and described below) and outer protrusions 162.

Arming mechanism 108 includes a handle 164, a first rim 166, a second rim 168, an internal groove 170 and an internal raised portion 172. Trigger mechanism 110 includes a body 174, a trigger button 176, a spring element 178 and a latch rim 180.

Launching mechanism 112 includes a lancet holder 182, a launch spring 184 (with launch spring first and second ends 186 and 188, respectively), and a retraction spring 190. Lancet holder 182 includes proximal end 192, a distal end 194, a first hollow portion 196, a second hollow portion 198, a first surface 200, a second surface 202, a space 204, an outwardly expandable portion 206, a slit 208, a lip 210, a cam surface 212, a depression 214, an internal surface 216, and a projection 218 (with a projection end 220).

Having introduced various components of compact lancing device 100, details of the interaction and functioning of such components will now be described with reference to FIGs. 1 through 6. A portion of arming mechanism 108 is visible to a user through arming member orifice 120 of housing 102 (see FIGs. I and 6 in particular). Handle 164 of arming mechanism 108 protrudes through arming member orifice 120 near proximal end 114 of housing 102 and on housing first surface 118. A user slides handle 164 to arm compact lancing device 100.

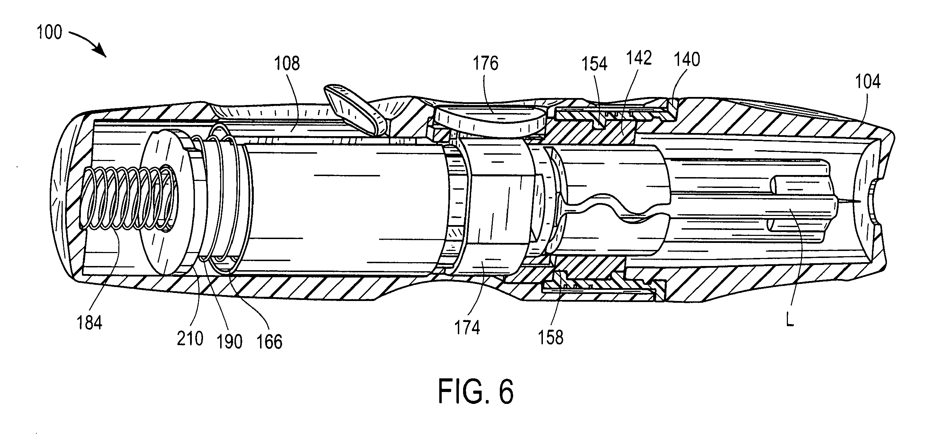

Trigger member 110 is accessible to a user through housing trigger member orifice 122 on housing first surface 118 (see, in particular, FIGs. 1 and 6). Housing trigger member orifice 122 is in close proximity to, and on the same housing surface (i.e., housing first surface 118) as, housing arming member orifice 120 in order that a user can operate both trigger mechanism 110 and arming mechanism 108 using one hand.

As depicted in FIG. 1, a lancing depth setting 150 (i.e., the numeral "6" in FIG. 1) is visible to a user through housing window 124 near housing distal end 116 on housing first surface 118. In addition, housing 102 includes a gripping feature 128 on second surface 126 of housing 102. Second surface 126 and gripping feature 128 are in oppositional relationship to handle 164 and trigger button 176 such that a user can easily grip and operate compact landing device 100 with one hand.

In the embodiment of FIG. 1, gripping feature 128 is an indentation in housing second surface 126. However, once apprised of the present disclosure, those skilled in the art will recognize that gripping feature 128 can take any suitable form including, but not limited to, one or more protrusions or recesses on the surface of housing 102.

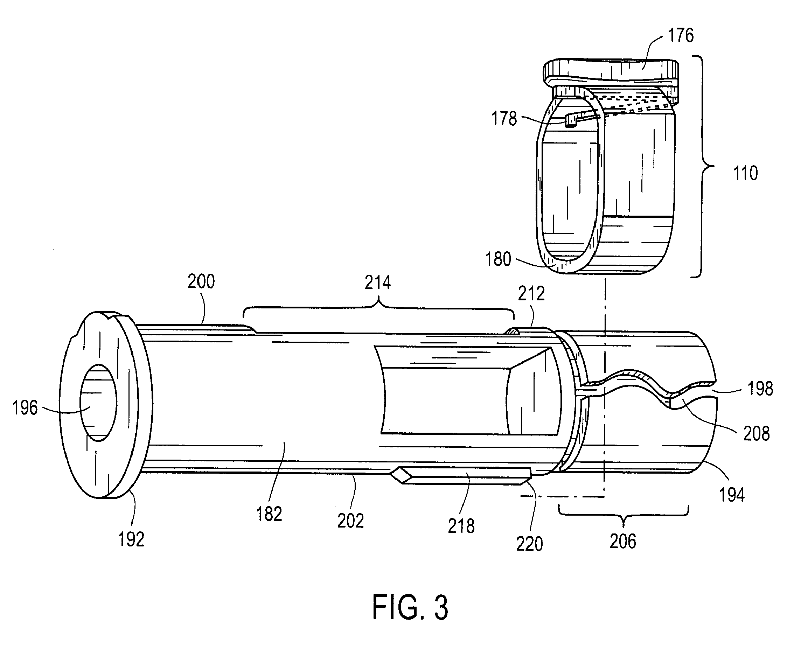

Lancet holder 182 is generally cylindrical in shape with first hollow portion 196 and second hollow portion 198 disposed at the proximal and distal ends 192 and 194, respectively, of lancet holder 182 (see, for example, FIG. 3). First hollow portion 196 extends into lancet holder 182 approximately a third of the distance from proximal end 192 to distal end 194. Second hollow portion 198 extends into lancet holder 182 approximately a third of the distance from distal end 194 to proximal end 192. Launch spring 184 is located at least partially within first hollow portion 196 and lancet L is removably retained at least partially within second hollow portion 198.

Space 204 of lancet holder 182 is approximately centrally located and bounded by first and second surfaces 200 and 202, respectively. Distal end 194 includes a radially and outwardly expandable portion 206 with a slit 208 configured such that lancet L (e.g., a suitable commercially available lancet) can easily be inserted into and removed from lancet holder 182.

Proximal end 192 includes a lip 210 that engages with first rim 166 of arming mechanism 108 during latching of lancet holder 182. Lip 210 further holds retraction spring 190 in surrounding relationship to lancet holder proximal end 192. First surface 200 includes a cam surface 212 adjacent to a depression 214 for a spring element 178 of trigger mechanism 110 to cooperate with (e.g., to react against). Second surface 202 includes an elongate projection 218 that, along with cam surface 212 and depression 214, function during arming and triggering operations, as will be described below.

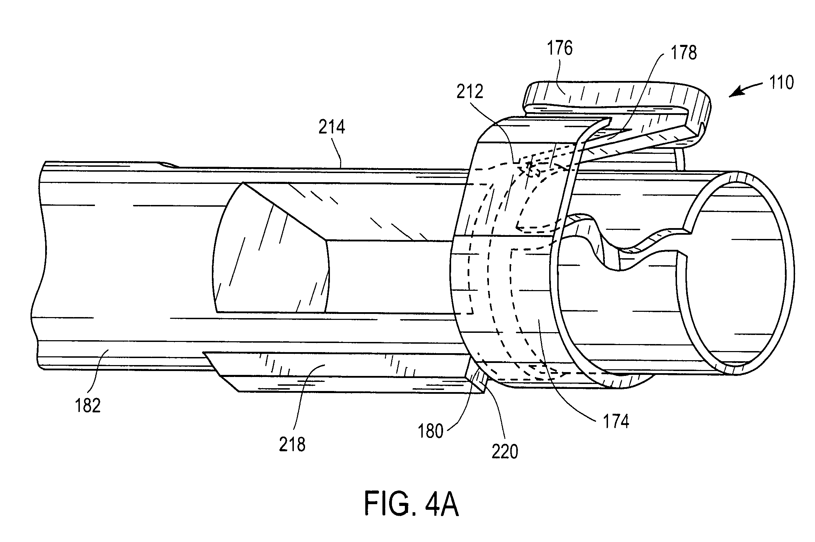

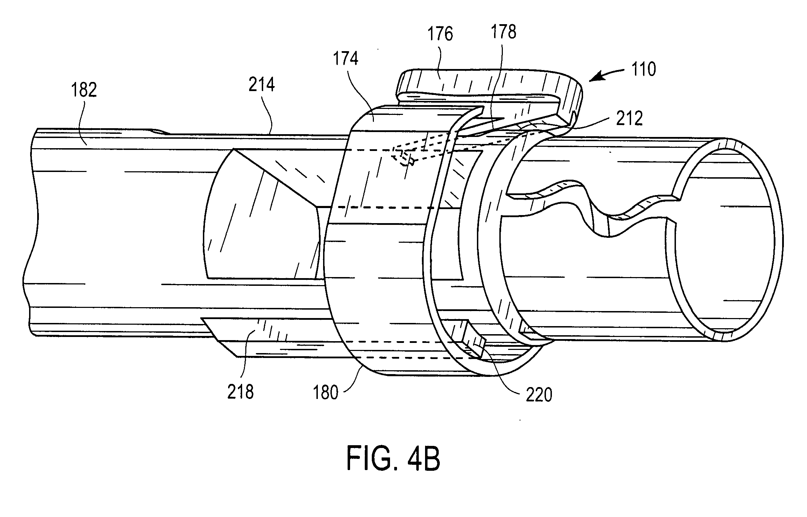

Launch spring 182 is configured to control movement of lancet holder 182. Launch spring first end 186 engages an internal surface of housing proximal end 114 and launch spring second end 188 engages an internal surface 216 of lancet holder 182 (see FIGS. 4A and 4B). Launch spring 184 typically applies a spring force to lancet holder 182 during launch of lancet L in the range of from about 0.25 pounds to 2 pounds and preferably from about 0.5 pounds to 1 pounds. In the embodiment of FIG. 1, retraction spring 190 is essentially concentric with launch spring 184, thereby contributing to the compactness of compact lancing device 100.

Retraction spring 190 resides substantially within the circumferential space between arming mechanism 108 and lancet holder 182. Retraction spring 190 pulls lancet holder 182 back after lancet needle N has been launched into a target site, dampens vibrations from lancet holder 182 during use of compact lancing device 100 and prevents lancet needle N from penetrating the target site a second time. Retraction spring 190 also returns arming mechanism 108 to a rest position after latching. One end of retraction spring 190 is also engaged by arming mechanism 108 during arming of compact lancing device 100, as will be described below. Retraction spring 190 can be formed of any suitable material including plastic materials (such as polypropylene and polyester) metal materials or any combinations thereof.

Arming mechanism 108 is generally hollow and elongate and is disposed in surrounding relation to lancet holder 182. Internal raised portion 172 of arming mechanism 108 engages one end of retraction spring 190 during arming of compact lancing device 100, as will be described below.

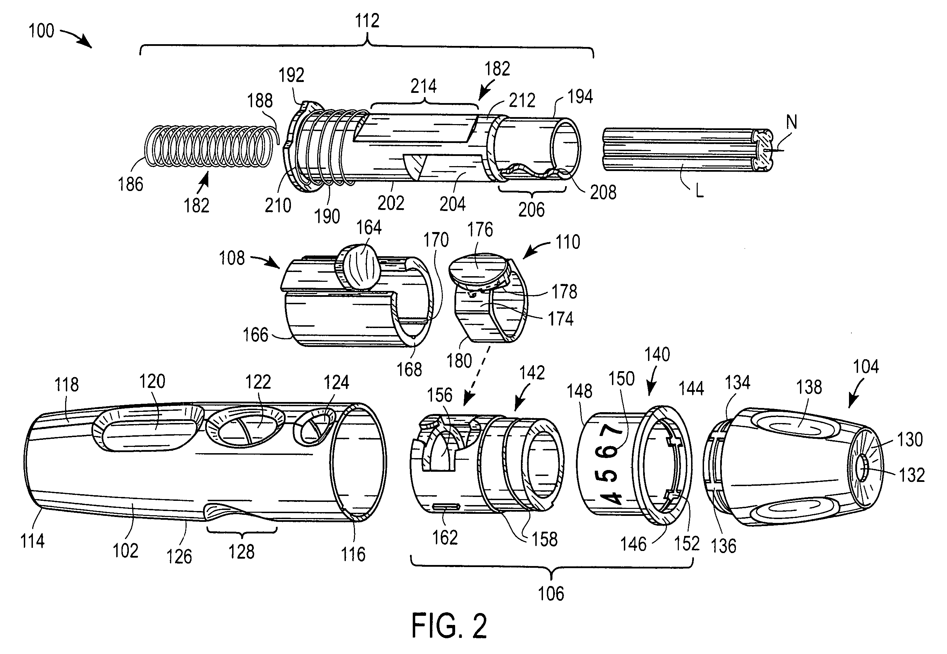

Lancet holder projection 218 is adapted to slidably move within internal groove 170 (see FIG. 2). Internal groove 170 engages lancet holder projection 218. Internal groove 170, therefore, limits relative rotational motion of lancet holder 182 upon actuation of compact lancing device 100, thereby reducing vibration and pain felt by the user. Internal groove 170 and guide member groove 160 are aligned to one another such that both internal groove 170 and guide member groove 160 can engage projection 218 simultaneously.

Trigger mechanism 110 is generally internally elongate, ring-shaped and disposed in a surrounding relationship to lancet holder 182. Trigger mechanism 110 can move laterally but not longitudinally relative to housing 102.

Spring element 178 projects into body 174 of trigger mechanism 110. Spring element 178 engages cam surface 212 when compact lancing device 100 is armed (see FIG. 3B) and slidably engages depression 157 when lancet 132 is fired as lancet holder 140 moves toward housing distal end 106 (see FIG. 4A). In both the armed and fired position of lancet holder 182, spring element 178 is at a minimal load while retaining an armed or loaded position, but is momentarily loaded to a greater extent when trigger button 176 is pressed to unlatch lancet holder 182. Therefore, the typical load on spring element 178 is low (i.e., less than 20 grams) even when lancing compact lancing device 100 is armed, thus improving the durability of compact lancing device 100. Trigger mechanism 110 can be formed (e.g., molded) in one piece, thus reducing the number of parts and simplifying the manufacture of compact lancing device 100.

When compact lancing device 100 is armed, trigger member latch rim 180 engages projection end 220 of lancet projection 218 and trigger button 176 moves laterally to a triggering position (as depicted in FIG. 4A). When lancet L is fired (i.e., when trigger button 176 is depressed), latch rim 180 slides over lancet projection 218, allowing lancet holder 182 to move toward housing distal end 116 (see FIG. 4B). Arming and triggering of compact lancing device 100 are described in more detail below with respect to FIGs. 7A through 7E.

Adjustment mechanism 106 enables a user to predetermine a depth of needle penetration into a target site. End cap 104 includes opening 132 for lancet needle N to pass through and a plurality of indentations 138 such that a user can grip end cap 104 and rotate, tip or pull the end cap away from housing 103 when replacing lancets. End cap 104 can be formed of any suitable material including, but not limited to, elastomeric materials such as rubber, latex or silicone such that when end cap 104 is removed, end cap 104 can optionally deform inward and grab onto lancet L, thereby allowing lancet L to be removed along with end cap 104.

Collar engaging end 134 of end cap 104 is configured to mate with collar first end 144. Collar engaging end 134 includes a plurality of raised features 136 for engaging with a plurality complimentary recesses 152 of collar 140. Raised features 136 and complimentary recesses 152 provide for detent-based retention of end cap 104, yet facilitate easy removal of end cap 104 by, for example, i.e., tipping to one side or pulling off.

Collar 140 includes an internal spiral thread 154 that engages a complimentary cam surface 158 (which is essentially an external spiral thread cam surface) on guide member 142, an rim 146 on first end 144, and a second end 148. Collar 140 is slidably moveable relative to housing 102 and guide member 142 and has a generally hollow cylindrical shape.

Guide member 142 includes an aperture 156 through which trigger member 110 is inserted such that trigger member 110 is nested therein. Guide member 142 further includes a groove 160 (see FIG. 7A-7C) that cooperates with lancet holder projection 218. Guide member groove 160 beneficially limits rotational motion of lancet holder 182 during firing of lancet L, thus reducing vibration and pain felt by the user.

Guide member 142 is held stationary relative to lancet holder 182 by attaching guide member 142 to the inner surface of housing 102 via outer protrusions 162 that mate with recesses (not shown) on the inner surface of housing 102. However, any attachment means known to those skilled in the art can be used to secure guide member 142 to housing 102 including, but not limited to a pin, a screw, and ultrasonic welding.

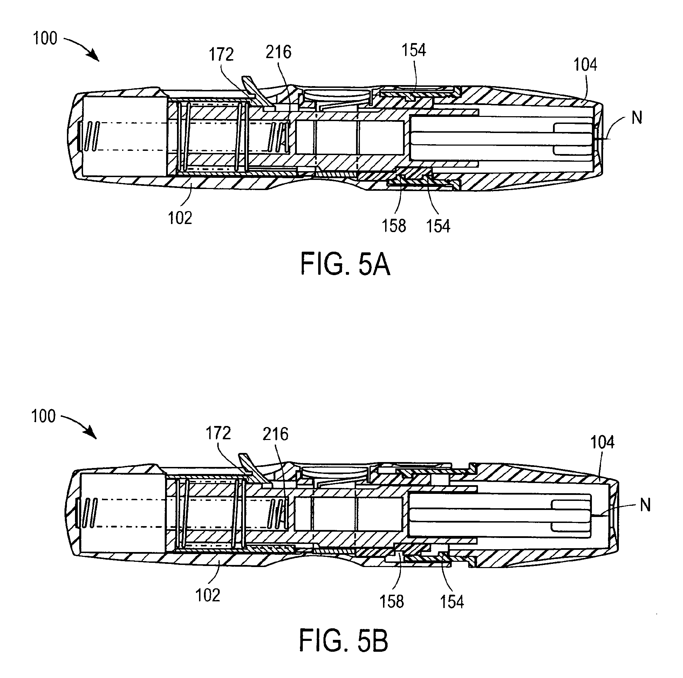

Rotation of end cap 104 adjusts the depth of needle penetration. When end cap 104 is rotated, raised features 136 engage with collar recesses 152 via a spline interface methodology. This causes collar internal spiral threads 154 to engage guide member cam surface 158, thereby moving end cap 104 away from or toward housing 102 and changing the distance needle N penetrates into a target site. In this regard, FIGs. 5A and 5B show cross-sectional views of lancing device 100 set at maximum and minimum needle penetration depth, respectively.

Arming mechanism 108, trigger mechanism 110, lancet holder 182, collar 140, and guide member 142 can, for example, be formed of rigid materials including, but not limited to, polycarbonate, polyester, polystyrene, polyamide, polyacetal, polyimide, polyketone, polyurethane polybutyleneteraphthalate or combinations thereof. Arming mechanism 108, trigger mechanism 110, lancet holder 182, collar 140, and guide member 142 can optionally contain lubricating additives including, for example, Teflon or graphite to reduce friction (and resulting vibration) therebetween.

All mechanisms of compact lancing device 100 cooperate with a close fit, thereby minimizing vibration during firing of lancet L. In addition, the interface between lancet holder lip 210 and arming mechanism first rim 166 can include materials that dampen sound and vibration (e.g., rubber or elastomeric materials). Such a sound/vibration dampening material interface can be formed, for example, as an additional component (e.g., a layer of sound/vibration dampening material) disposed between lip 210 and first rim 166 or by forming lip 210 and/or first rim 166 either partially or completely of a sound and/or vibration dampening material. Use of tight tolerances (such as tight tolerances in the range of 0.0005 inches to 0.003 inches) and sound/vibration dampening materials may reduce the perception of pain by the device user.

Referring to FIGs. 7A through 7E, the operation of compact lancing device 100 (depicted in simplified cross-section in FIG. 7A) will be described. In compact lancing device 100, trigger mechanism 110 and arming mechanism 108 are located on the same side of the compact lancing device such that arming and triggering can be performed with one hand.

Compact lancing device 100 is armed by moving arming mechanism 108 toward proximal end 114 of housing 102 such that one end of retraction spring 190 is engaged by internal raised portion 172 of arming mechanism 108 and the other end of retraction spring 190 with lip 210 of lancet holder 182 (see FIG. 7B). When first rim 166 contacts lip 210, lancet holder 182 starts to move towards proximal end 114. As retraction spring 190 is thus engaged, lancet holder 182 moves toward housing proximal end 114 and spring element 178 of trigger mechanism 110 engages cam surface 212 on lancet holder 182. Furthermore, trigger member latch rim 180 engages projection end 220 of a lancet holder projection 218 and trigger mechanism 110 moves laterally relative to housing 102 such that trigger button 176 is raised above (or alternatively level with) first surface 118 of the housing 102, thus giving the user a visual and tactical indication of when compact lancing device 100 is armed (see FIG. 7B).

When arming mechanism 108 is released, both arming mechanism 108 and the end of retraction spring 190 in contact with first rim 166 move back to their original positions (see FIG. 7C). Arming of compact lancing device 100 can also be achieved by an axial force exerted on lancet holder 182 by an act of inserting a lancet into lancet holder 182.

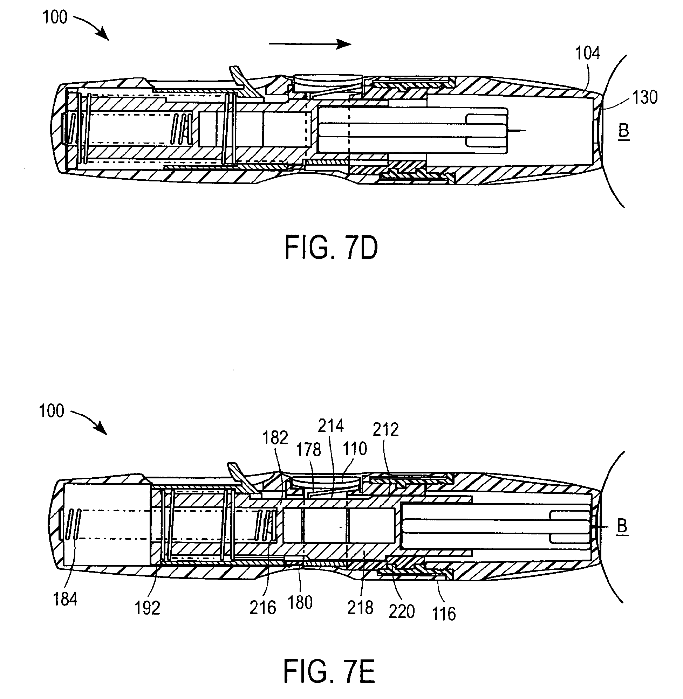

Next, end cap engaging surface 130 is contacted with a target site. Subsequently, the engaging surface is urged towards the target site, thereby creating a bulge B in the target site (see FIG. 7D, where the arrow indicates the direction of urging).

When trigger mechanism 110 is triggered (e.g., trigger button 176 is pressed in a lateral direction relative to housing 102), latch rim 180 is disengaged from projection end 220 of lancet holder 182, thereby allowing projection 218 to slide over latch rim 180 and lancet holder 182 to move toward end cap 104 (see FIG. 7E). Simultaneously, spring element 178 disengages from lancet holder cam surface 212 and then engages with depression 214 in the surface of lancet holder 182 and moves toward housing distal end 116 (see FIG. 7E). Launch spring 184 exerts a force on lancet holder internal surface 216 causing lancet holder 182 to move longitudinally toward bulge B (i.e., to be fired or launched toward bulge B) such that bulge B is lanced with the lancet needle N.

As described above, trigger mechanism 110 has a role in both the arming and triggering of compact lancing device 100, thereby eliminating the need and expense of separate components to perform these functions.

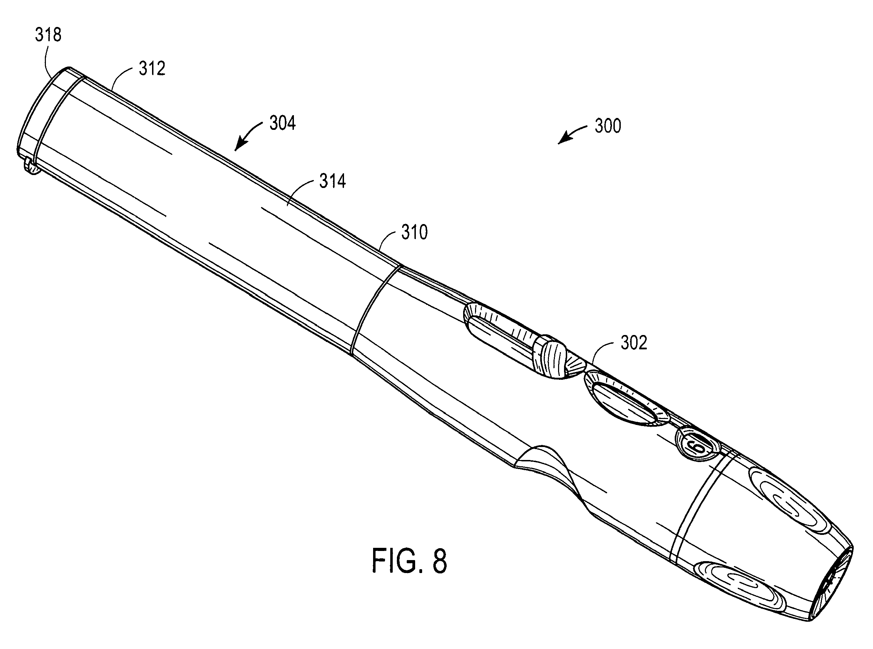

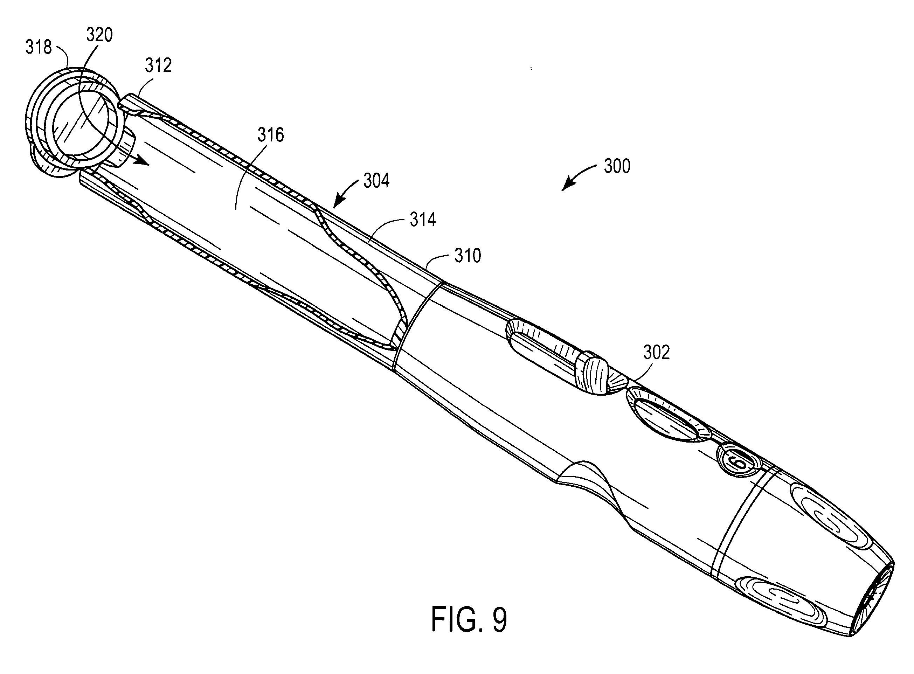

FIGs. 8 and 9 depicts a combined lancing and auxiliary device 300 according to an embodiment of the present invention. Combined lancing and auxiliary device 300 includes lancing device 302 (e.g., compact lancing device 100 of FIG. 1) and a lancing auxiliary device, namely a storage compartment 304, attached thereto. Storage compartment 304 is configured to receive and removably retain items (not shown) related to the use of lancing device 302. Items related to lancing device 302 that can be removably retained in storage compartment 304 include, but are not limited to, lancets, test strips, pharmaceuticals and alcohol swabs. Storage compartment 304 is optionally configured to receive and removably retain items (not shown) unrelated to the use of lancing device 302. Items unrelated to lancing device 302 that can be removably retained in storage compartment 304 include, but are not limited to, coins, pills and medications.

For illustrative purposes only in FIGs. 8 and 9, lancing device 302 of combined lancing and auxiliary device 300 is illustrated as compact lancing device 100 of FIG. 1. However, once apprised of the current invention, those skilled in the art will recognize that lancing device 302 of combined lancing and auxiliary device 300 can be any suitable lancing device.

Storage compartment 304 is generally elongate and cylindrical in shape and includes a proximal end 310, a distal end 312, a body 314 and a cavity 316 (see FIG. 9) for storing items related to the use of lancing device 302. Storage compartment 304 can be removably attached to lancing device 302 by, for example, slide mounting, snap fitting or screw fitting techniques. Compartment proximal end 310 can also be fixedly (i.e., permanently) attached to housing 302 by, for example welding or molding techniques.

Distal end 312 of compartment 304 includes a cap 318 and an opening 320 (see FIG. 9) for inserting and removing items from compartment 304. Cap 318 retains and protects items contained in compartment 304 from possible contamination or damage due to exposure to humidity or light. Cap 318 can be attached to compartment distal end 312 by, for example, a snap-type fit (with or without a hinge connection) or a screw fit.

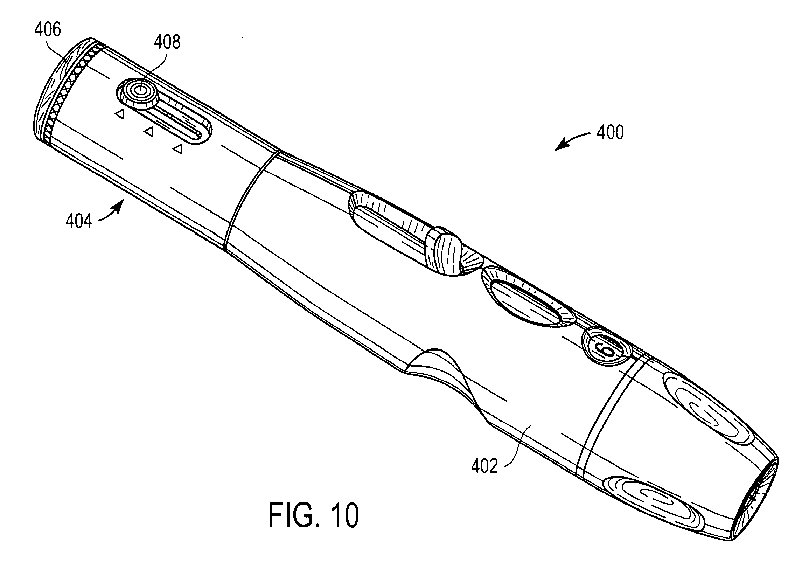

FIG. 10 depicts a combined lancing and auxiliary device 400 according to another embodiment of the present invention. Combined lancing and auxiliary device 300 includes lancing device 402 (e.g., compact lancing device 100 of FIG. 1) and a lancing auxiliary device, namely an illumination device 404, attached thereto. In this embodiment, illumination device 404 includes an illuminating means 406 (e.g., a flashlight) for illuminating a target site and a sliding switch 408 for adjusting the intensity of illuminating means 406.

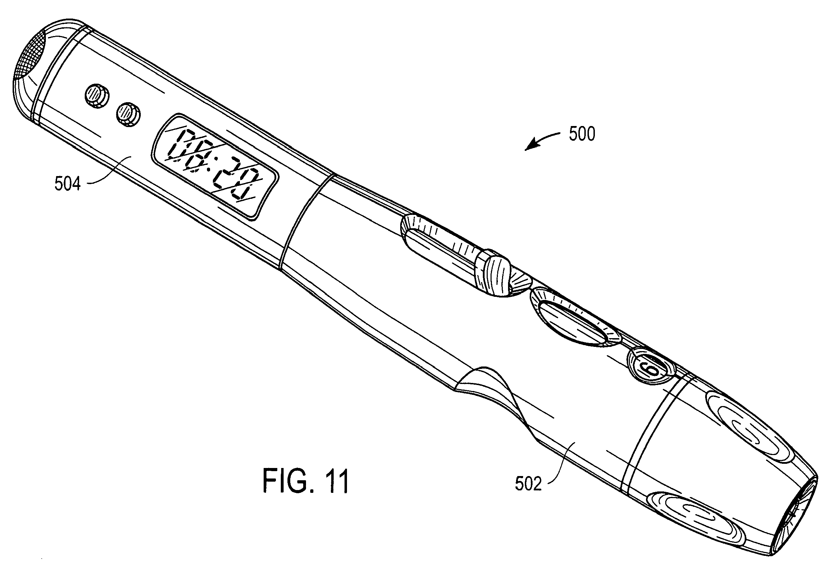

FIG. 11 depicts a combined lancing and auxiliary device 500 according to yet another embodiment of the present invention. Combined lancing and auxiliary device 500 includes lancing device 502 (e.g., compact lancing device 100 of FIG. 1) and a lancing auxiliary device, namely a timing and sound recording device 504, attached thereto.

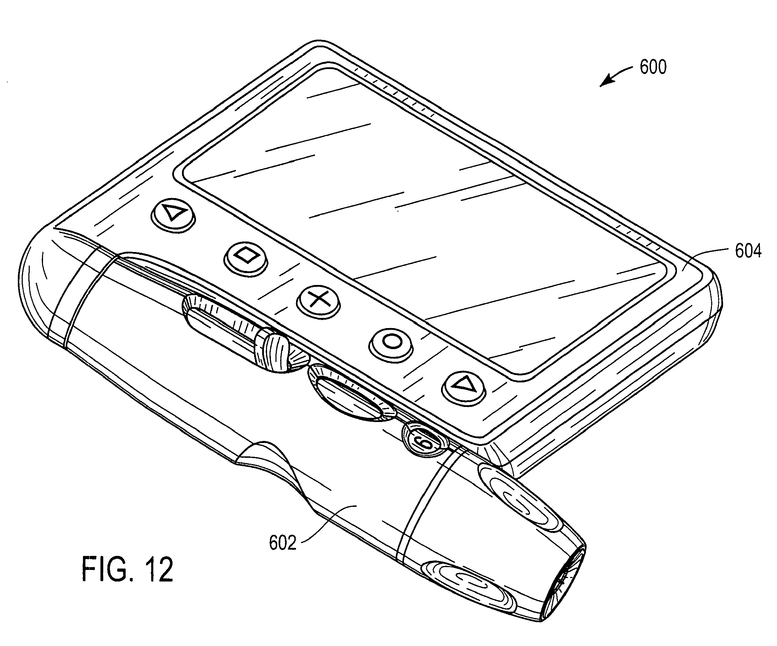

FIG. 12 depicts a combined lancing and auxiliary device 600 according to yet another embodiment of the present invention. Combined lancing and auxiliary device 600 includes lancing device 602 (e.g., compact lancing device 100 of FIG. 1) and a lancing auxiliary device, namely a personal digital assistant device 604, attached thereto. It is envisioned that personal digital assistant device 604 can be used to aid in the management of a medical condition (e.g., diabetes) by, for example, storing glucose testing results, carbohydrate counting, calculating insulin dosage based on food consumed or recording the date and time of testing. Personal digital assistant device 604 can be either removably or permanently attached to lancing device 602.

It should be understood that various alternatives to the embodiments of the invention described herein may be employed in practicing the invention. It is intended that the following claims define the scope of the invention and that structures within the scope of these claims and their equivalents be covered thereby.

## Claims

1. A combined lancing and auxiliary device comprising:
a lancing device; and
a lancing auxiliary device attached to the lancing device.

2. The combined lancing and auxiliary device of claim 1, wherein the lancing auxiliary device is permanently attached to the lancing device.

3. The combined lancing and auxiliary device of claim 1, wherein the lancing auxiliary device is detachably attached to the lancing device.

4. The combined lancing and auxiliary device of claim 1, wherein the auxiliary device is a compartment for securely and removably retaining items.

5. The combined lancing and auxiliary device of claim 4, wherein the compartment is configured for securely and removably retaining items related to the use of the lancing device.

6. The combined lancing and auxiliary device of claim 5, wherein the compartment is configured for securely and removably retaining at least one of lancets and test strips.

7. The combined lancing and auxiliary device of claim 1, wherein the auxiliary device is an illumination device.

8. The combined lancing and auxiliary device of claim 1, wherein the auxiliary device is a timing device.

9. The combined lancing and auxiliary device of claim 1, wherein the auxiliary device is a personal digital assistant device.

10. The combined lancing and auxiliary device of claim 1, wherein the auxiliary device is any combination of an illumination device, a timing device, a personal digital assistant device and a compartment for securely and removably retaining items related to the use of the lancing device.

11. The combined lancing and auxiliary device of claim 1, wherein the lancing device includes:
a housing;
a moveable lancet holder disposed within the housing and configured to hold a lancet;
a launching mechanism disposed within the housing, the launching mechanism including:
a launch spring; and
a retraction spring
an arming mechanism disposed partially within the housing; and
a trigger mechanism disposed partially within the housing,
wherein the lancet holder, launching mechanism, arming mechanism and trigger mechanism are operatively connected to lance a target site with a lancet held by the moveable lancet holder, and
wherein the launch spring and retraction spring are configured in an essentially concentric arrangement.
